## Europäisches Patentamt
## European Patent Office
## Office européen des brevets

(19)

(11) Veröffentlichungsnummer: **0 229 375 B1**

(12) **EUROPÄISCHE PATENTSCHRIFT**

(45) Veröffentlichungstag der Patentschrift: **11.09.91**

(51) Int. Cl.5: **A61K 7/16**

(21) Anmeldenummer: **86117913.3**

(22) Anmeldetag: **22.12.86**

Die Akte enthält technische Angaben, die nach dem Eingang der Anmeldung eingereicht wurden und die nicht in dieser Patentschrift enthalten sind.

(54) **Mittel zur oralen Hygiene.**

(30) Priorität: **07.01.86 DE 3600165**

(43) Veröffentlichungstag der Anmeldung:
**22.07.87 Patentblatt 87/30**

(45) Bekanntmachung des Hinweises auf die Patenterteilung:
**11.09.91 Patentblatt 91/37**

(84) Benannte Vertragsstaaten:
**AT BE CH DE ES FR GB GR IT LI LU NL SE**

(56) Entgegenhaltungen:
**EP-A- 0 038 868**
**EP-A- 0 049 830**

(73) Patentinhaber: **Blendax GmbH**
**Rheinallee 88**
**W-6500 Mainz 1(DE)**

(72) Erfinder: **Afseth, John, Prof. Dr.**
**Frognerstervn 7 D**
**0387 Oslo 8(NO)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 229 375 B1

## Beschreibung

Die vorliegende Erfindung betrifft ein Mittel zur oralen Hygiene, insbesondere ein Zahn- und Mundpflegemittel, mit einer die Bildung von Zahnbelag hemmenden Wirksamkeit, das als belagshemmende Wirkstoffe ein Gemisch aus Kupferverbindungen und Hexetidin enthält.

Die Verwendung von Hexetidin, 1,3-Bis(2-ethylhexyl)-5-amino-5-methylhexahydropyrimidin, als Antiseptikum, unter anderem zur Therapie von Entzündungen im Mund- und Rachenbereich, ist bereits seit langem bekannt.

Es wurde auch bereits versucht, Hexetidin als Mittel zur Verhinderung der Bildung von Zahnbelag einzusetzen, jedoch konnte eine einschlägige Wirkung nicht nachgewiesen werden, so daß in der Fachwelt die Meinung bestand, daß Hexetidin enthaltende Zahn- und Mundpflegemittel zur Hemmung der Zahnbelagsbildung nicht geeignet sind.

Es ist bekannt, daß das Kupfer-Kation eine die Bildung von Zahnbelag hemmende Wirkung ausübt, wenn entsprechende Lösungen von Kupferverbindungen topisch mit den Zähnen in Berührung gebracht werden (AADR-Abstracts 1975, Nr. 117; Caries Research, Vol. 18, 1984, S. 434 - 439).

Diese Kupferionen liefernde Verbindungen weisen die Nachteile, wie sie für andere belagshemmende Verbindungen auf Basis antimikrobieller Stoffe, wie beispielsweise quaternären Ammoniumverbindungen oder Chlorhexidinsalzen, bekanntgeworden sind, insbesondere die Verfärbung der Zähne bei Langzeitanwendung, nicht auf.

In den EP-A 38 867 und 38 868 sind daher bereits Zahnpasta-Zusammensetzungen beschrieben, die Kupferverbindungen in bestimmter Zusammensetzung enthalten.

Es wurde nunmehr überraschenderweise gefunden, daß diese Wirkung der Kupferverbindungen gegen Zahnbelag noch gesteigert werden kann, wenn man solchen Zusammensetzungen Hexetidin zusetzt.

Es handelt sich dabei offensichtlich um einen unerwarteten synergistischen Effekt. Das Auftreten dieses Effektes ist umso überraschender, als, wie bereits oben ausgeführt, Hexetidin alleine praktisch keine die Bildung von Zahnbelag hemmende Wirkung aufweist, so daß nicht vorhersehbar war, daß die an sich bereits bekannte einschlägige Wirkung von Kupferionen durch Zusatz dieser für sich nicht aktiven Substanz noch gesteigert werden könnte.

Aus der EP-A 49,830 sind bereits Mittel zur Inhibierung von Zahnbelag bekannt, die eine Kombination aus Hexetidin und Zinksalzen enthalten. Von Kupfersalzen ist dabei jedoch keine Rede, es wird im Gegenteil auf Seite 9 im letzten Absatz darauf hingewiesen, daß es sich um einen spezifischen Effekt handelt, und der Ersatz von Zink durch andere Metallsalze diesen Effekt aufheben würde. Es bestand daher für den Fachmann keinerlei Anlaß, in Abkehr von diesem Vorurteil den Einsatz von Kupfersalzen ausstelle von Zinksalzen in Erwägung zu ziehen.

Die Menge an Kupferverbindungen, die in den erfindungsgemäßen Mitteln zur oralen Hygiene zum Einsatz gelangt, sollte so bemessen sein, daß 0,001 bis 1,5 Gew.-% Cu, berechnet auf die gesamte Zusammensetzung, anwesend sind.

Der bevorzugte Mengenbereich liegt dabei bei 0,01 bis 1,0 %, insbesondere 0,025 bis 0,1 % Cu.

Als Kupferionen liefernde Verbindungen sind prinzipiell alle toxikologisch unbedenklichen, schleimhautverträglichen, auch noch einigermaßen wasserlöslichen Kupferverbindungen geeignet.

Von den anorganischen Salzen seien beispielhaft genannt:

Kupferchlorid, $CuCl_2$, und dessen Dihydrat; Kupferfluorid $CuF_2$, und dessen Dihydrat; Kupferfluorsilikat, $(CuSiF_6)$, und dessen Hexahydrat; Kupfersulfat, $(CuSO_4)$, und dessen Pentahydrat; Kupfernitrat bzw. dessen Tri- und Hexahydrat sowie auch ausgefallenere Kupfersalze wie Bromide, Bromate, Chlorate, Jodate, Fluorphosphate.

Bevorzugte Kupfersalze organischer Säuren sind das Acetat, Formiat, Benzoat, Citrat, Tartrat, Lactat, Malat, Mandelat, Sorbat, Pantothenat, Gluconat, Phytat, Glycerophosphat, Cinnamat, Butyrat, Propionat, Laurat, Oxalat, Salicylat.

Geeignet sind auch die Kupfersalze von Aminosäuren wie Glycinat oder Glutamat. Ein besonders bevorzugtes Aminosäuresalz ist das Kupferaspartat.

Das Hexetidin ist in den erfindungsgemäßen Mittel einer Menge zwischen 0,025 und 0,5, insbesondere 0,05 und 0,25 Gew.-%, bezogen auf die Gesamtzusammensetzung, enthalten.

Aus den im folgenden beschriebenen Vergleichsversuchen ergibt sich die überlegene Wirksamkeit einer das erfindungsgemäße Gemisch enthaltenden Zusammensetzung gegenüber Mundspülungen, die in analogen Konzentrationen nur jeweils eine der Substanzen, nämlich Hexetidin und Kupfersulfat, enthalten.

20 Tage alte Osborne-Mendel-Ratten wurden in 7 Gruppen mit je 16 Versuchstieren aufgeteilt und erhielten die Plaque-Standard-Kost 2000 F.

Zu Versuchsbeginn werden die Tiere mit 0,1 ml einer standardisierten Bakteriensuspension von

2

Actinomyces viscosus OMZ 105 geimpft. Eine Nachimpfung erfolgt jeweils in einwöchigem Abstand.

Die Behandlung der Versuchstiere mit den zu untersuchenden Präparaten beginnt am 23. Lebenstag, je zweimal täglich werden mit einer Spritze 0,1 ml Testpräparat/Versuchstier appliziert. Nach 4 Wochen wurden die Tiere getötet und die Plaquebildung an den beiden ersten Bukkalflächen und den ersten 4 Lingualflächen der Molaren 1 und 2 im Oberkiefer beurteilt; d.h., 12 Begutachtungsflächen/Tier.

Die Bewertung erfolgt nach Anfärbung mit Erythrosin-Lösung nach dem folgenden Schema:

0: Keine Plaque

1: Bis zu 1/3 der Fläche mit Plaque überzogen.

2: Bis zu 2/3 der Fläche mit Plaque überzogen.

3: Mehr als 2/3 der Fläche mit Plaque überzogen.

Die Punktzahl wird addiert und ein Mittelwert ($\bar{x}$) gebildet.

### Ergebnis

| Gruppe | $\bar{x}$ | $\sigma$ |
|---|---|---|
| 1 | 22,38 | +/- 2,42 |
| 2 | 11,56 | +/- 4,02 |
| 3 | 19,63 | +/- 2,19 |
| 4 | 8,50 | +/- 3,33 |
| 5 | 21,06 | +/- 3,26 |

Zusammensetzung der untersuchten Präparate:

Gruppe 1: 2%-iges Hydroxyethylcellulose-Gel (Placebo).

Gruppe 2: 2%-iges Hydroxyethylcellulose-Gel mit 0,2 % $CuSO_4 . 2H_2O$. (= 0,05 % Cu).

Gruppe 3: 2%-iges Hydroxyethylcellulose-Gel mit 0,05 % Hexetidin.

Gruppe 4: 2%-iges Hydroxyethylcellulose-Gel mit 0,20 % Kupfersulfat $5H_2O$ (= 0,05 % Cu) und 0,05% Hexetidin.

Gruppe 5: Unbehandelte Kontrolle.

Diese Ergebnisse beweisen mit statistisch gesicherter Signifikanz die Überlegenheit der erfindungsgemäßen synergistischen Kombination aus Kupferverbindungen und Hexetidin.

Die erfindungsgemäßen Mittel zur oralen Hygiene können in jeder beliebigen Applikationsform vorliegen. Bevorzugt werden die Applikationsformen Zahnpasta, wobei es sich um eine opake oder um eine gelförmige transparente Zahnpasta handeln kann, Mundwasser und Kaugummi; jedoch ist auch jede andere Applikationsform wie beispielsweise Mundspray, Lutsch- oder Kautablette oder Zahnpulver für diesen Zweck geeignet.

Eine Zahnpasta kann opak oder eine durch Verwendung geeigneter, in ihren Brechungsindices mit dem Brechungsindex des Trägermaterials übereinstimmender Poliermittel hergestellte transparente Zahnpasta sein.

Geeignete Poliermittel sind beispielsweise Alkalialuminiumsilikate wie solche vom Zeolith-Typ A, beschrieben in den EP-A-. 2690 und 3023, verschiedene Calciumphosphate wie Dicalciumorthophosphat in Form seines Dihydrats oder wasserfrei, Calciumcarbonat, Aluminiumoxid und dessen Trihydrate, insbesondere α-Aluminiumoxidtrihydrat, Tricalciumphosphat, Calciumpyrophosphat, unlösliche Alkalimetaphosphate, Siliciumdioxide verschiedener Modifikationen wie SiliciumdioxidXerogele, - Hydrogele oder gefällte Siliciumdioxide, oder pulverförmige Kunststoffe wie Polymethylmethacrylat mit einer Korngrößenverteilung zwischen etwa 0,5 und etwa 5 µm enthalte.

Es können selbstverständlich auch Poliermittelgemische aus den genannten Substanzen eingesetzt werden, beispielsweise ein Gemisch aus α-Aluminiumoxidhydrat und/oder Calciumcarbonat und synthetischem Zeolith A im Verhältnis von etwa 1:1.

Der Poliermittelanteil in den erfindungsgemäßen Zahnpasten liegt vorzugsweise zwischen etwa 20 und 60 Gew.-% der Gesamtzusammensetzung.

Es ist selbstverständlich möglich, die üblichen in Zahnpasten eingesetzten oberflächenaktiven Verbin-

EP 0 229 375 B1

dungen in Mengen bis zu etwa 2,5 Gew.-% der Gesamtzusammensetzung zu verwenden.

Solche synthetischen oberflächenaktiven Stoffe sind beispielsweise Alkylsulfate, Alkylethersulfate Olefinsulfonate, Natriumlauroylsarcosinat oder ampholytische, nichtionische oder kationaktive Verbindungen oder auch Seifen wie beispielsweise solche von Laurinsäure, Myristinsäure, Palmitinsäure, Stearinsäure oder Gemischen derselben, beispielsweise Cocosölfettsäuren oder Talgfettsäuren.

Eine Übersicht über in Zahnpasten einsetzbare Zusammensetzungen findet sich, wie überhaupt über sonstige zur Herstellung von Zahnpflegemitteln üblicherweise zum Einsatz gelangende Stoffe und die dabei angewandten Herstellungsverfahren, in dem Handbuch von M.S. Balsam und E. Sagarin, "Cosmetics - Science and Technology", 2nd Ed., Vol. 1, S. 423 bis 533 (1972), auf das hier ausdrücklich Bezug genommen wird.

Gleiches gilt hinsichtlich der in Zahnpasten üblicherweise in Mengen zwischen 10 und 35 Gew.-% zum Einsatz gelangenden Feuchthaltemittel, wie Glycerin, Diole wie 1,4-Butandiol oder 1-2-Propandiol oder Zuckeralkohole wie Sorbit, Mannit oder Xylit und Polyglykole mit niederen Molekulargewichten, ebenso für Verdickungsmittel.

Bevorzugte Verdickungsmittel sind Carboxymethylcellulose und deren Alkalisalze, insbesondere Natriumcarboxymethylcellulose, Hydroxyalkylcellulosen wie Hydroxymethylcellulose und Hydroxyethylcellulose, Methylcellulose, Pflanzengummen wie Tragant, Gummi arabicum Carayagummi, Guargummi, Xanthangummi und Irish Moos, synthetische Polyelektrolyt wie die Alkalisalze der Polyacrylsäure sowie anorganische Verdikkungsmittel, beispielsweise kolloidales Magnesiumaluminiumsilikat oder disperses Siliciumoxid, deren Mengenanteil in Zahnpasten zwischen 0,25 und 5 Gew.-% der Gesamtzusammensetzung liegt.

In den erfindungsgemäßen Mitteln zur oralen Hygine können selbstverständlich auch weitere Wirkstoffe Verwendung finden. Solche sind insbesondere die bekannten kariesprophylaktischen Fluoride, vorzugsweise in einer solchen Menge, daß die Konzentration an reinem Fluor im Mittel 0,05 bis 1 Gew.-%, vorzugsweise 0,1 bis 0,5 Gew.-% des Mittels beträgt.

Geeignete Fluorverbindungen sind insbesondere die verschiedenen Salze der Monofluorphosphorsäure wie Natrium-, Kalium-Lithium-, Calcium- und Aluminiummono- und difluorphosphat sowie verschiedene, Fluor in ionisch gebundener Form enthaltene Fluoride, insbesondere Alkalifluoride wie Natrium-, Lithium-, Kalium- und Ammoniumfluorid, Zinnfluorid, Manganfluorid, Kupferfluorid, Zirkoniumfluorid und Aluminiumfluorid sowie Gemische oder Anlagerungsproduke dieser Fluoride untereinander und mit anderen Fluorverbindungen, beispielsweise Alkalimanganfluoride.

Weitere, in den erfindungsgemäßen Mitteln zur oralen Hygiene einsetzbare Stoffe sind Zahnbelag entfernende Substanzen, beispielsweise Zinksalze, Mittel zur Verhinderung von Zahnsteinbildung wie Hydroxyethan-1,1-diphosphonsäure oder Alkylendiaminotetramethylenphosphonsäuren und deren wasserlösliche Salze, Allantoin, Azulen, etc..

Im folgenden werden einige Beispiele gegeben, die das Wesen der vorliegenden Erfindung charakterisieren:

4

Beispiel 1

Zahnpasta

| | |
|---|---|
| α-Aluminiumoxidtrihydrat | 58,50 (Gew.-%) |
| (Korngrößenverteilung etwa 1-15 μm) | |
| Sorbitlösung (70 %) | 25,50 |
| Xanthum-Gum | 0,60 |
| Natriummonofluorphosphat | 0,80 |
| Saccharin-Natrium | 0,10 |
| Konservierungsmittel | 0,30 |
| Natriumlaurylsulfat | 0,40 |
| Aromagemisch | 0,10 |
| Kupferaspartat | 0,26 |
| Hexetidin | 0,05 |
| Wasser | ad 100,00 |

Beispiel 2

Zahnpasta

| | |
|---|---|
| Synthetisches Zeolith A | 24,00 (Gew.-%) |
| $(Na_{12}(AlO_2)_{12}(SiO)_2)_{12}\cdot 27\ H_2O$ | |
| Dicalciumorthophosphat | 10,00 |
| Carboxymethylcellulose | 1,20 |
| Natriumlaurylsulfat | 2,00 |
| Glycerin | 6,00 |
| Sorbit | 15,00 |
| Konservierungsmittel | 0,30 |
| Aromagemisch | 1,00 |
| Pyrogene Kieselsäure | 1,55 |
| Saccharin-Natrium | 0,05 |
| Natriummonofluorphosphat | 0,80 |
| Kupferaspartat | 0,25 |
| Hexetidin | 0,08 |
| Wasser | ad 100,00 |

### Beispiel 3

Zahnpasta

| | |
|---|---|
| Irish Moos | 0,50 (Gew.-%) |
| Xanthan-Gum | 0,50 |
| Glycerin | 7,50 |
| Sorbit | 22,00 |
| Kupferformiat . $4H_2O$ | 0,30 |
| Kupferfluorid ($CuF_2$) | 0,25 |
| Natriumlauroylsarcosinat | 1,40 |
| Gehärtetes Melamin-Formaldehyd-Kondensat (mittlerer Teilchendurchmesser ~1-10 µm) | 28,50 |
| Titandioxid | 0,50 |
| Saccharin-Natrium | 0,10 |
| Aromagemisch | 1,00 |
| p-Hydroxybenzoesäuremethylester | 0,10 |
| p-Hydroxybenzoesäure-n-propylester | 0,05 |
| Hexetidin | 0,22 |
| Entsalztes Wasser | 37,30 |

### Beispiel 4

Zahnpasta

| | |
|---|---|
| Irish Moos | 0,50 (Gew.-%) |
| Xanthan-Gum | 0,50 |
| Glycerin | 7,50 |
| Sorbit | 28,00 |
| Kupfersulfat.5 $H_2O$ | 0,25 |
| Hexetidin | 0,10 |
| Natriumlauroylsarcosinat | 1,40 |
| Gefällte Kieselsäure (vom Typ Sident®3) | 22,50 |
| Titandioxid | 0,50 |
| Saccharin-Natrium | 0,10 |
| Aromagemisch | 1,00 |
| p-Hydroxybenzoesäuremethylester | 0,10 |
| p-Hydroxybenzoesäure-n-propylester | 0,05 |
| Entsalztes Wasser | ad 100,00 |

Beispiel 5

Zahnpasta

| | |
|---|---|
| Xanthan-Gum | 1,20 (Gew.-%) |
| Glycerin | 15,00 |
| Sorbit | 12,00 |
| Kupfersalicylat $(Cu(C_7H_5O_3)_2 \cdot 4\ H_2O)$ | 1,00 |
| Hexetidin | 0,08 |
| Siliciumdioxid-Xerogel | 16,00 |
| (vom Typ Syloid® AL 1, Oberfläche | |
| etwa 800 m²/g) | |
| Pyrogenes Siliciumdioxid (vom Typ | 3,00 |
| Aerosil®) | |
| Titandioxid | 0,50 |
| Aromagemisch | 1,00 |
| Saccharin-Natrium | 0,16 |
| Trinatriumcitrat | 0,25 |
| p-Hydroxybenzoesäureethylester | 0,20 |
| Entsalztes Wasser | ad 100,00 |

## Beispiel 6

Zahnpasta

| | |
|---|---|
| Glycerin | 19,00 (Gew.-%) |
| Sorbit (70%ig) | 7,00 |
| Polyethylenglykol 300 | 3,00 |
| Kupferlactathydrat | 1,20 |
| Zinnfluorid ($SnF_2$) | 0,40 |
| Hydroxyethan-1,1-diphosphonsäure, Trinatriumsalz | 1,25 |
| Bromchlorophen | 0,05 |
| Hexetidin | 0,06 |
| Benzoesäure | 0,15 |
| Dehydracetsäure | 0,10 |
| p-Hydroxybenzoesäure-n-propylester | 0,05 |
| Polymethylmethacrylatpulver (mittlerer Teilchendurchmesser 3-8 µm | 20,00 |
| Siliciumdioxid-Xerogel (vom Typ Syloid® 70, Oberfläche etwa 290 m²/g) | 8,50 |
| Pyrogenes Siliciumdioxid (vom Typ Aerosil®) | 1,20 |
| Nariumlaurylethersulfat (25%ig in Ethanol) | 10,00 |
| Xanthan-Gum | 0,80 |
| Entsalztes Wasser | ad 100,00 |

## Beispiel 7:

### Kaugummi:

| | |
|---|---|
| Gummibase | 2,10 |
| Sorbit | 25,00 |
| Xylit | 20,00 |
| Saccharin-Natrium | 0,30 |
| Hexetidin | 0,30 |
| Kupfersulfat . 5 $H_2O$ | 0,50 |
| Glycerin | 2,00 |
| Aromagemisch | 3,70 |
| Ascorbinsäure | 1,00 |
| Fructose | 15,00 |

## Beispiel 8:

### Mundwasser-Konzentrat:

| | |
|---|---|
| Aromagemisch | 5,00 (Gew.-%) |
| Kupferaspartat | 2,50 |
| Zinkcitrat . 2 $H_2O$ | 0,25 |
| Hexetidin | 0,35 |
| Nichtionischer Emulgator | 1,80 |
| n-Propanol | 5,00 |
| 1-Methoxypropanol(-2) | 35,00 |
| Glycerin | 8,50 |
| Phenylsalicylat | 0,55 |
| Saccharin-Natrium | 0,30 |
| Wasser | ad 100,00 |

Das Konzentrat wird vor Gebrauch im Verhältnis von etwa 1:4 mit Wasser verdünnt.

## Beispiel 9:

### Kaugummi:

| | |
|---|---|
| Gummibase | 1,80 |
| Sorbit | 25,00 |
| Xylit | 20,00 |
| Saccharin-Natrium | 0,30 |
| Kupferfluorid | 0,50 |
| Kupfersulfat . 5 $H_2O$ | 1,30 |
| Hexetidin | 0,50 |
| Glycerin | 2,00 |
| Aromagemisch | 2,70 |
| Ascorbinsäure | 1,00 |
| Fructose | 15,00 |

## Beispiel 10:

### Gebrauchsfertige Mundspülung:

| | |
|---|---|
| Kupfersulfat . 5 $H_2O$ | 0,100 (Gew.-%) |
| Hexetidin | 0,075 |
| Ethanol | 5,000 |
| Glycerin | 2,500 |
| Nichtionischer Emulgator | 0,500 |
| Farbstoff, Aroma | q.s. |
| Wasser | ad 100,000 |

Die zweimal tägliche Anwendung dieser Zusammensetzung brachte im klinischen Doppelblindversuch nach 5 Tagen eine signifikante Reduktion des Plaque-Indexes nach Silness-Löe, verglichen mit der Wirksamkeit von Lösungen, die jeweils nur eine der beiden Komponenten in entsprechenden Konzentrationen enthielten.

**Patentansprüche**

1. Mittel zur oralen Hygiene, dadurch gekennzeichnet, daß es ein synergistisch wirkendes Gemisch aus Kupferionen liefernden Verbindungen in einer Menge von 0,001 bis 1,5 Gew.-% Cu und 0,025 bis 0,5 Gew.-% Hexetidin, jeweils berechnet auf die Gesamtzusammensetzung, enthält.

2. Mittel nach Anspruch 1, dadurch gekennzeichnet, daß es die Kupferionen liefernden Verbindungen in einer Menge von 0,01 bis 1,0 % Cu, berechnet auf die Gesamtzusammensetzung, enthält.

3. Mittel nach Anspruch 1, dadurch gekennzeichnet, daß es die Kupferverbindungen in einer Menge von

0,025 bis 0,1 Gew.-% Cu, berechnet auf die Gesamtzusammensetzung, enthält.

4. Mittel nach Anspruch 1, dadurch gekennzeichnet, daß es das Hexetidin in einer Menge zwischen 0,05 und 0,25 Gew.-%, bezogen auf die Gesamtzusammensetzung, enthält.

**Claims**

1. Agent for oral hygiene, characterised in that it contains a synergistically acting mixture of copper ion-yielding compounds in a quantity of 0.001 to 1.5% by weight of Cu and 0.025 to 0.5% by weight of hexetidine, based on the total composition in each case.

2. Agent according to claim 1, characterised in that it contains the copper ion-yielding compounds in a quantity of 0.01 to 1.0% of Cu, based on the total composition.

3. Agent according to claim 1, characterised in that it contains the copper compounds in a quantity of 0.025 to 0.1% by weight of Cu, based on the total composition.

4. Agent according to claim 1, characterised in that it contains the hexetidine in a quantity of between 0.05 and 0.25% by weight, with respect to the total composition.

**Revendications**

1. Produit pour l'hygiène buccale, caractérisé en ce qu'il contient un mélange à effet synergique d'un composé générateur d'ions cuivre en une quantité de 0,001 à 1,5% en poids de Cu et de 0,025 à 0,5% en poids d'hexétidine, calculés chacun par rapport à la composition totale.

2. Produit selon la revendication 1, caractérisé en ce qu'il contient les composés générateurs d'ions cuivre en une quantité de 0,01 à 1,0% de Cu, calculée par rapport à la composition totale.

3. Produit selon la revendication 1, caractérisé en ce qu'il contient les composés du cuivre en une quantité de 0,025 à 0,1% en poids de Cu, calculée. par rapport à la composition totale.

4. Produit selon la revendication 1, caractérisé en ce qu'il contient l'hexétidine en une quantité comprise entre 0,05 et 0,25% en poids, calculée par rapport à la composition totale.